# EUROPÄISCHE PATENTSCHRIFT

(11) **EP 0 926 123 B1**
(45) Veröffentlichungstag und Bekanntmachung des Hinweises auf die Patenterteilung: **14.11.2001**
(21) Anmeldenummer: 98123787.8
(22) Anmeldetag: 15.12.1998
(51) Int. Cl.: C07C 45/86, C07C 47/02

(54) **Verfahren zur Stabilisierung von Aldehyden**
Process for the stabilization of aldehydes
Procédé pour la stabilisation d'aldéhydes

(30) Priorität: 23.12.1997 DE 19757531
(43) Veröffentlichungstag der Anmeldung: 30.06.1999
(73) Patentinhaber: Celanese Chemicals Europe GmbH, 60439 Frankfurt am Main (DE)
(72) Erfinder: Riedel, Michael Dr., Bay City TX 77414 (US); Zgorzelski, Wolfgang, 46147 Oberhausen (DE); Messerschmidt, Michael, 46539 Dinslaken (DE); Bergrath, Klaus, 46147 Oberhausen (DE)

(56) Entgegenhaltungen:
- US-A- 2 170 625
- US-A- 4 020 109
- CHEMICAL ABSTRACTS, vol. 073, no. 9, 31. August 1970 Columbus, Ohio, US; abstract no. 044900, SATO M: "Stabilization of isobutyraldehyde" XP002095945 & JP 45 012282 - (MITSUBISHI CHEMICAL INDUSTRIES CO., LTD.)
- DATABASE WPI Section Ch, Week 8529 Derwent Publications Ltd., London, GB; Class B05, AN 85-174961 XP002095946 & JP 60 104058 A (NIPPON SODA CO) , 8. Juni 1985

## Beschreibung

Die vorliegende Erfindung betrifft ein Verfahren zur Stabilisierung von Aldehyden gegen Polymerisation und Autokondensation:

Aldehyde neigen aufgrund ihrer großen Reaktivität zur Polymerisation und Autokondensation. Bei der Polymerisation bilden sich vorwiegend trimere Produkte. Im Falle von Isobutyraldehyd entsteht beispielsweise 2,4,6-Triisopropyl-1,3,5-trioxan, doch auch bei anderen aliphatischen Aldehyden mit 3 - 14 Kohlenstoffatomen kommt es durch Polymerisation zur Bildung des cyclischen Trimeraldehyds (Trialkyltrioxan). Die Trimerisierung wird durch den Einfluß chemischer Substanzen wie Chlor oder Brom, Phosphorpentoxid, Schwefelsäure, Schwefelwasserstoff, Chlorwasserstoff, Fluorwasserstoff, Bortrifluorid, Aluminiumchlorid oder Zinkchlorid katalysiert. Unter dem Einfluß solcher aciden Verbindungen setzt die Polymerisation der Aldehyde spontan ein. Bei ausreichend hoher Konzentration der aciden Verbindung kommt es somit innerhalb weniger Minuten zur Bildung kristalliner Trimeraldehyde. Bei Konzentrationen von bis zu 10 ppm der aciden Verbindung läuft die Trimerenbildung etwas langsamer innerhalb einiger Tage ab. Darüber hinaus begünstigen tiefe Temperaturen, d.h. Temperaturen von etwa 0°C oder darunter, oder UV-Licht die Polymerisation der aliphatischen Aldehyde. Ein weiteres Problem stellt die Neigung der Aldehyde dar, unter Einwirkung alkalischer Substanzen der Aldolkondensation zu unterliegen.

Wegen des Überganges in derartige höhermolekulare Verbindungen können Aldehyde nicht beliebig lange Zeit aufbewahrt werden. Zwar zerfallen die Polymerisations- und Autokondensationsprodukte der Aldehyde bei höherer Temperatur wieder, doch steht ihre Bildung einer uneingeschränkten technischen Verwendung der Aldehyde entgegen. Man ist daher bemüht, die Entstehung höhermolekularer Produkte aus den Aldehyden zu verhindern. Über einen begrenzten Zeitraum ist dies möglich, indem man die Aldehyde in hochreiner Form herstellt und lagert. Die hierzu erforderlichen Reinigungsoperationen sind jedoch so aufwendig, daß sie sich für eine kommerzielle Herstellung der Aldehyde verbieten.

Es ist nun bekannt, daß die Polymerisations- und Autokondensationsreaktionen durch Zusatz bestimmter Stoffe unterbunden werden können. An diese Stoffe stellt die Praxis eine Reihe Forderungen, die erfüllt sein müssen, falls der Aldehyd ohne Einschränkung den verschiedensten Anwendungsfällen zugeführt werden soll. Hierzu gehört, daß die betreffende Substanz bereits in niedriger Konzentration lange Zeit hindurch wirksam sein muß und darüber hinaus bei der Verarbeitung des Aldehyds durch chemische Umsetzungen nicht stört.

Als Stabilisatoren für Isobutyraldehyd sind z.B. Mercaptobenzimidazol und 2,2-Methylen-di-(4-methyl-6-tert. butylphenol) beschrieben. Diese Stabilisatoren sind jedoch nur eine unzureichend lange Zeit wirksam. So wird gemäß DE-OS- 29 05 267 bei Zusatz von 100 ppm Mercaptobenzimidazol zu Isobutyraldehyd bereits nach einer Lagerzeit des stabilisierten Aldehyds von nur 5 Wochen wieder ein erhebliches Maß der Trimerisierung beobachtet.

Nach einem anderen Verfahren setzt man den Aldehyden zur Verhinderung der Polymerisation eine Losung von Diphenylamin in Ethanol zu. Diese Arbeitsweise stellt aber ebenfalls nicht sicher, daß die Polymerisation über einen längeren Zeitraum unterdrückt wird.

Aus der DE-OS-29 05 267 sowie der DE-OS 29 17 789 ist es bekannt, daß man Isobutyraldehyd sowie andere aliphatische Aldehyde mit 3-14 Kohlenstoffatomen durch den Zusatz von Triethanolamin oder Dimethylethanolamin gegen Polymerisation und Autokondensation stabilisieren kann. Bei Verwendung dieser Stabilisatoren kann bereits mit relativ geringen Konzentrationen eine gute Stabilisierung über einen längeren Zeitraum bewirkt werden. Beschrieben wird beispielsweise, daß mit 10 ppm der genannten Ethanolamine, bezogen auf den Aldehyd, die Bildung hochmolekularer Verbindungen durch Polymerisation und Autokondensation, z.B. bei Einwirkung von Sauerstoff, über einen Zeitraum von 30 Wochen ausgeschlossen werden kann. Beim Zusatz von 20-100 ppm, bezogen auf den Aldehyd, unterbinden die Stabilisatoren die Bildung des Trimeren bzw. des Aldolkondensationsproduktes bei der Lagerung des Aldehyds ohne besondere Vorkehrungen über einen Zeitraum von etwa einem Jahr. Nachteilig an diesen Stabilisatoren ist jedoch, daß sie sich nur mit einem erheblichen destillativen Aufwand wieder von den Aldehyden abtrennen lassen.

Nach der Lehre von JP 70 12,282 läßt sich die Trimerenbildung im Isobutyraldehyd zurückdrängen, wenn man dem Isobutyraldehyd eine alkalisch reagierende Substanz in einer Menge von 500 ppm zusetzt. Als alkalisch reagierende Substanzen kommen beispielsweise Na₂CO₃, K₂CO₃, (NH₄)₂CO₃, CaCO₃, Ca(OH)₂, AcONa, MgO, NaHCO₃ oder Natriumstearat, Magnesiumlaurat, Natriumsilikat oder Ammoniumhydroxid in Betracht.

Aus JP 45 012282 B4 ist das Problem der Stabilisierung von Isobutyraldehyd gegen die Bildung von Trimeren ebenfalls bekannt. Beschrieben wird, daß die Behandlung von Isobutyraldehyd mit einer wässrigen Alkalilösung keinerlei Wirkung zeigt. Eine Stabilisierung kann nur erreicht werden, wenn die alkalische Substanz dem Isobutyraldehyd als Feststoff zugesetzt wird oder in Form einer sehr konzentrierten wäßrigen Lösung, wobei in diesem Fall entscheidend ist, daß die Wassermenge unterhalb der Sättigungsgrenze des Isobutyraldehyds liegt. Als alkalische Verbindungen kommen Alkalimetallverbindungen (Carbonatsalze, Bicarbonatsalze, Silikate und Fettsäuresalze), Erdalkalimetallverbindungen (Oxide, Hydroxide, Carbonatsalze, Bicarbonatsalze und Fettsäuresalze) sowie Ammoniak bzw Ammoniumcarbonat zum Einsatz. Die zugesetzten Mengen der alkalischen Verbindung sind jedoch sehr groß. Im Fall von Natriumhydrogen-, Natrium-, Kalium-, Ammonium-, Calciumcarbonat sowie Calciumhydroxid, Natriumessigsäure und Magnesiumoxid als Stabilisatoren werden dem Isobutyraldehyd jeweils 500 ppm zugesetzt, eine Menge, die im Zusammenhang der JP 45 012282 B4 bereits als gering angesehen wird. Durch den Einsatz solch hoher Mengen des alkalisch reagierenden Stabilisators wird zwar die Trimerenbildung unterbunden, Probleme durch die zunehmend einsetzende alkalikatalysierte Aldolkondensation des Isobutyraldehyds sind jedoch unausweichlich. Hinzukommt, daß der Zusatz der alkalischen Substanz als Feststoff zu größeren, z.B. in Tanks gelagerten Aldehydmengen mit dem Problem verbunden ist, eine vollständige Auflösung, Dispergierung sowie gleichmäßige Verteilung der alkalischen Substanz im gesamten Aldehydvolumen zu erzielen.

Aufgabe der Erfindung ist es daher, ein verbessertes Verfahren bereitzustellen, mit dem für einen möglichst langen Zeitraum Polymerisations- und Autokondensations-reaktionen der Aldehyde ausgeschlossen werden können.

Gelöst wird diese Aufgabe durch ein Verfahren, mit dem aliphatische C₃-C₁₄-Aldehyde durch den Zusatz von alkalisch reagierenden Substanzen stabilisiert werden, welches dadurch gekennzeichnet ist, daß als alkalisch reagierende Substanz Alkalimetallhydroxide, Erdalkalimetallhydroxide, Alkalimetallcarbonate, Erdalkalimetallcarbonate, Alkalimetallcarboxylate oder Erdalkalimetallcarboxylate dem zu stabilisierenden Aldehyd in Mengen von 0.05-20 ppm, bevorzugt 0.05-5 ppm, besonders bevorzugt 0.05-2,5 ppm, bezogen auf den Aldehyd, zugesetzt werden.

Das erfindungsgemäße Verfahren zeichnet sich dadurch aus, daß die eingesetzten Stabilisatoren bereits in extrem geringen Konzentrationen wirksam sind. Schon 0.05 ppm des Stabilisators schließen die Bildung hochmolekularer Verbindungen durch Polymerisation oder Aldolkondensation bei Lagerung des Aldehyds, selbst bei niedrigen Temperaturen, ohne weitere Vorkehrungen über einen Zeitraum von mehreren Wochen aus. Hervorzuheben ist außerdem, daß die zur Stabilisierung der Aldehyde eingesetzten Substanzen die Weiterverarbeitung der Aldehyde nicht stören. Ist eine Abtrennung der alkalischen Substanz vor der Weiterverarbeitung der Aldehyde dennoch erwünscht, so ist dies durch eine einfache Destillation möglich, bei der die alkalische Substanz im Destillationssumpf verbleibt. Besonders bemerkenswert ist, daß die Stabilisatoren, obwohl sie alkalisch reagieren, bei den Aldehyden keine Aldolkondensationsreaktion auslösen.

Als Alkalimetallhydroxide werden bevorzugt Natrium- oder Kaliumhydroxid, und als Erdalkalimetallhydroxide bevorzugt Calciumhydroxid eingesetzt. Als Alkalimetallcarbonate kommen bevorzugt Natrium- und Kaliumcarbonat und als Erdalkalimetallcarbonate bevorzugt Magnesium- und Calciumcarbonat zum Einsatz. Als Alkalimetallcarboxylat wird insbesondere Natriumbutyrat verwendet.

Die alkalischen Substanzen werden üblicherweise als 0.01 bis 1M, bevorzugt 0.05 bis 0.5 M und insbesondere 0.1 bis 0.25 M wäßrige Lösung eingesetzt. In Einzelfällen kann es sich auch als zweckmäßig erweisen, die alkalischen Substanzen, insbesondere die Alkalimetallcarboxylate und dabei bevorzugt das Natriumbutyrat, als Feststoffe zuzugeben.

Beispiele für Aldehyde, die nach dem erfindungsgemäßen Verfahren stabilisiert werden können, sind: Propanal, n- und i-Butanal, n- und i- Pentanal, n- und i-Hexanal, n- und i- Heptanal, n- und i-Octanal, n- und i- Nonanal, n- und i- Decanal, Undecanal, Dodecanal, Laurinaldehyd, Methylnonylaldehyd (MNA), Tridecylaldehyd sowie Myristylaldehyd.

Diese Aldehyde können bis zu 3 Gew%, bevorzugt 0.5 bis 2 Gew.% und insbesondere 0.75 bis 1.25 Gew.% Wasser enthalten.

Das erfindungsgemäße Verfahren kann ausgeführt werden, indem der Stabilisator in Form der wäßrigen Lösung vorgelegt wird und der gegebenenfalls ebenfalls wasserhaltige Aldehyd hinzugegeben wird. Umgekehrt kann die wässrige Lösung des Stabilisators auch zu dem gegebenenfalls wasserhaltigen Aldehyd gegeben werden.

### Beispiele:

### Beispiele 1-3:

Das zur Stabilisierung eingesetzte Natriumhydroxid wird als 0.1 M wäßrige Lösung in der entsprechenden Menge in Polyethylenflaschen vorgelegt, anschließend mit der entsprechenden Aldehydmenge versetzt und mit Stickstoff überlagert. Der Isobutyraldehyd in den Beispielen 1 und 2 enthält dabei bereits jeweils 2% Deionat (entsalztes Wasser) und der n-Butyraldehyd in Beispiel 3 1% Deionat.
Anschließend werden die Polyethylenflaschen für 20 Minuten mittels einer Rotiermaschine geschüttelt, um eine optimale Durchmischung zu erzielen. Im Fall von Beispiel 2 wird die Flasche während der gesamten Versuchsdauer von 4 Wochen geschüttelt.
Die Lagerung der Flaschen erfolgt unter Lichtausschluß über die jeweiligen Versuchszeiten. Die Probennahme nach den unterschiedlichen Lagerungszeiten wird jeweils unter Zugabe von 100 ppm Triethanolamin durchgeführt, um den aktuellen Zustand der jeweiligen Probe sicherzustellen. Die Analyse der Proben wird mittels Gaschromatographie vorgenommen. Alle Arbeiten werden unter Stickstoff durchgeführt.

**Tabelle 1**

| | Trimerenbildung [Gew%] | | | | | | | | |
|---|---|---|---|---|---|---|---|---|---|
| | Beispiel 1 | | | Beispiel 2 | | | Beispiel 3 | | |
| | i-Butyraldehyd | | | i-Butyraldehyd unter ständigem Schütteln | | | n-Butyraldehyd | | |
| NaOH Tage | Ohne | 0.17 ppm | 0.5 ppm | Ohne | 0.2 ppm | 2 ppm | Ohne | 0.1 ppm | 0.17 ppm |
| 0 | | 0.025 | | | 0.005 | | | 0.063 | |
| 7 | 0.305 | 0.169 | 0.048 | | | | 0.125 | 0.063 | 0.063 |
| 14 | | | | 1.611 | 0.284 | 0.085 | | | |
| 28 | | | | 2.317 | 0.434 | 0.087 | | | |

### Beispiele 4-10 :

Das zur Stabilisierung eingesetzte Natriumhydroxid, Kaliumhydroxid bzw. Natriumcarbonat wird als wäßrige Lösung mit der in Tabelle 2 angegebenen Konzentration und Menge in Polyethylenflaschen vorgelegt, anschließend mit der entsprechenden Menge an n-Butyraldehyd versetzt und mit Stickstoff überlagert.
Anschließend werden die Polyethylenflaschen für 20 Minuten mittels einer Rotiermaschine geschüttelt, um eine optimale Durchmischung zu erzielen.
Die Lagerung der Flaschen erfolgt unter Lichtausschluß über die jeweiligen Versuchszeiten. Die Probennahme nach den unterschiedlichen Lagerungszeiten wird jeweils unter Zugabe von 100 ppm Triethanolamin durchgeführt, um den aktuellen Zustand der jeweiligen Probe sicherzustellen. Die Analyse der Proben wird mittels Gaschromatographie vorgenommen. Alle Arbeiten werden unter Stickstoff durchgeführt.

**Tabelle 2:**

| Bsp | Zusatz | Analytik | Probenahme nach (h) Einsatz 48 h 120 h [Gew%] | | |
|---|---|---|---|---|---|
| 4 | 1 % Deionat | n-C₄-al Trim C₄-al K1 Trim C₄-al K2 Σ Tetramer Σ Aldol | 99,928 < 0,005 < 0,005 < 0,005 0,002 | 99,856 0,051 0,010 0,009 0.004 | 99,545 0,279 0,054 0,045 0,004 |
| 5 | 1 % Deionat + 0,17 ppm NaOH *(=0.0017 %-ige Lsg.)* | n-C₄-al Trim C₄-al K1 Trim C₄-al K2 Σ Tetramer Σ Aldol | 99,928 < 0,005 < 0,005 < 0,005 0,002 | 99,924 0,002 < 0,005 < 0,005 0,004 | 99,919 0,002 < 0,005 < 0,005 0,004 |
| 6 | 0.5 % Deionat + 0.17 ppm NaOH *(=0.0034 %-ige Lsg)* | n-C₄-al Trim C₄-al K1 Trim C₄-al K2 *Σ* Tetramer Σ Aldol | 99,928 < 0,005 < 0,005 < 0,005 0.002 | 99,921 < 0,005 < 0,005 < 0,005 0.004 | 99,914 < 0,005 < 0,005 < 0,005 0,007 |
| 7 | 0.25 % Deionat + 0.17 ppm NaOH *(=0.0068 %-ige Lsg.)* | n-C₄-al Trim C₄-al K1 Trim C₄-al K2 Σ Tetramer Σ Aldol | 99,928 < 0,005 < 0,005 < 0,005 0,002 | 99,918 0,002 < 0,005 < 0,005 0,004 | 99,907 0,002 < 0,005 < 0,005 0,007 |
| 8 | 1 % Deionat + 0.17 ppm KOH *(=0.0017 %-ige Lsg.)* | n-C₄-al Trim C₄-al K1 Trim C₄-al K2 Σ Tetramer Σ Aldol | 99.928 < 0,005 < 0,005 < 0,005 0,002 | 99.923 < 0,005 < 0,005 < 0,005 0,004 | 99,919 < 0,005 < 0,005 < 0,005 0,005 |
| 9 | 1 % Deionat + 500ppm Na₂CO₃ *(=4.8 %-ige Lsg.)* | n-C₄-al Trim C₄-al K1 Trim C₄-al K2 Σ Tetramer Σ Aldol | 99,928 < 0,005 < 0,005 < 0,005 0,002 | 99,888 < 0,005 < 0,005 < 0,005 0,039 | 99,869 < 0,005 < 0,005 < 0,005 0,056 |
| 10 | 1 % Deionat + 500 ppm NaOH *(=4.8 %-ige Lsg.)* | n-C₄-al Trim C₄-al K1 Trim C₄-al K2 Σ Tetramer Σ Aldo | 99,928 < 0,005 < 0,005 < 0,005 0,002 | spontan einsetzende Aldolisierung | |

Hierbei haben die Abkürzungen folgenden Bedeutungen:
- n-C₄-al :: n-Butyraldehyd
- Trim C₄-al K1 :: 2,4,5-Tri-n-propyl-1,3,5-trioxan in eee- oder aaa-Stellung
- Trim C₄-al K2 :: 2,4,6-Tri-n-propyl-1,3,5-trioxan in eea- oder aae-Stellung
- Σ Tetramer :: tetrameres Polymerisationsprodukt des n-Butyraldehyds
- Σ Aldol :: Summe der Aldolkondensationsprodukte

### Beispiele 11-14:

In allen Beispielen 11-14 wird zunächst der n-Butyraldehyd mit einem Wassergehalt von 1 Cew% durch Zusatz von Schwefelsäure auf einen Säuregehalt von 1 ppm eingestellt. Bei Beispiel 11 handelt es sich um eine Blindprobe, der feine alkalisch reagierende Substanz als Stabilisator zugegeben wird. In den Beispielen 12 und 13 werden anschließend 20 ppm bzw. 10 ppm festes Natriumbutyrat und in Beispiel 14 10 ppm Natriumhydroxid zum n-Butyraldehyd zugegeben. Die mit Stickstoff überlagerte Flasche wird anschließend über 20 Minuten mittels einer Rotiermaschine geschüttelt, um eine optimale Durchmischung zu gewährleisten. Die Lagerung der Flaschen erfolgt unter Lichtausschluß über die jeweiligen Versuchszeiten. Die Entnahme von jeweils 250 mL Proben nach den unterschiedlichen Lagerungszeiten wird jeweils unter Zugabe von 100 ppm Triethanolamin durchgeführt, um den aktuellen Zustand der jeweiligen Probe sicherzustellen. Alle Arbeiten werden unter Stickstoff durchgeführt. Die Analyse der Proben wird mittels Gaschromatographie vorgenommen.

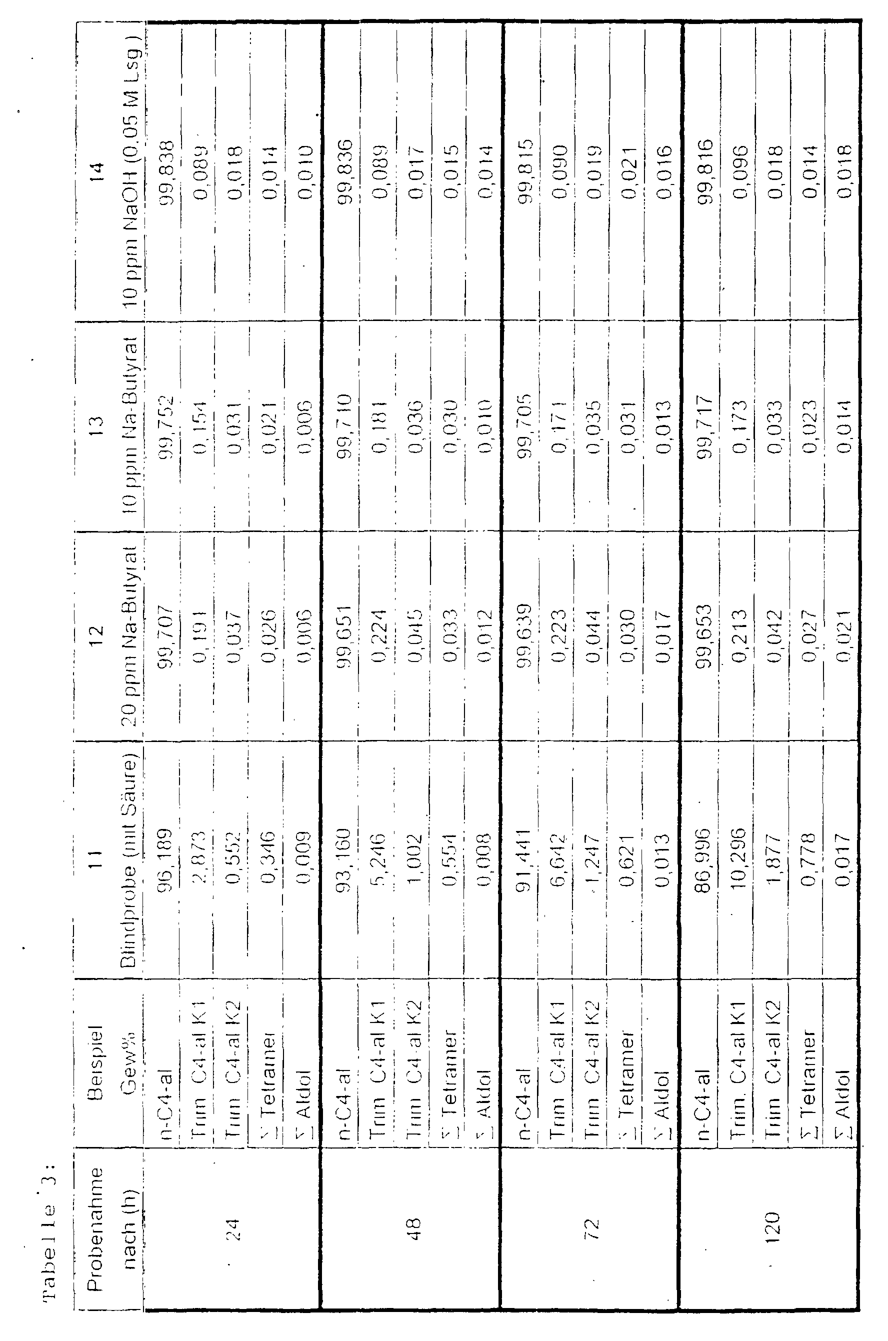

### Beispiele 15-18:

Bei Beispiel 15 handelt es sich um eine Blindprobe, der keine alkalisch reagierende Substanz als Stabilisator zugegeben wird. In den Beispielen 16,17 und 18 werden anschließend 0.5 ppm festes Natriumbutyrat, festes Calciumbutyrat sowie Natriumhydroxid als 0.05 M Lösung zum n-Butyraldehyd zugegeben. Die mit Stickstoff überlagerte Flasche wird anschließend über 20 Minuten mittels einer Rotiermaschine geschüttelt, um eine optimale Durchmischung zu gewährleisten. Die Lagerung der Flaschen erfolgt unter Lichtausschluß über die jeweiligen Versuchszeiten. Die Entnahme von jeweils 250 mL Proben nach den unterschiedlichen Lagerungszeiten wird jeweils unter Zugabe von 100 ppm Triethanolamin durchgeführt, um den aktuellen Zustand der jeweiligen Probe sicherzustellen. Alle Arbeiten werden unter Stickstoff durchgeführt. Die Analyse der Proben erfolgt mittels Gaschromatographie.

### Beispiele 19-21:

Bei Beispiel 19 handelt es sich um eine Blindprobe, der keine alkalisch reagierende Substanz als Stabilisator zugegeben wird. In den Beispielen 20 und 21 werden anschließend 0.5 ppm bzw. 0.25 ppm Natriumhydroxid als 0.1 M Lösung zum n-Butyraldehyd zugegeben. Die mit Stickstoff überlagerte Flasche wird anschließend über 20 Minuten mittels einer Rotiermaschine geschüttelt, um eine optimale Durchmischung zu gewährleisten. Die Lagerung der Flaschen erfolgt unter Lichtausschluß über die jeweiligen Versuchszeiten. Die Entnahme von jeweils 250 mL Proben nach den unterschiedlichen Lagerungszeiten erfolgt jeweils unter Zugabe von 100 ppm Triethanolamin, um den aktuellen Zustand der jeweiligen Probe sicherzustellen. Alle Proben werden unter Stickstoff durchgeführt. Die Analyse der Proben erfolgt mittels Gaschromatographie.

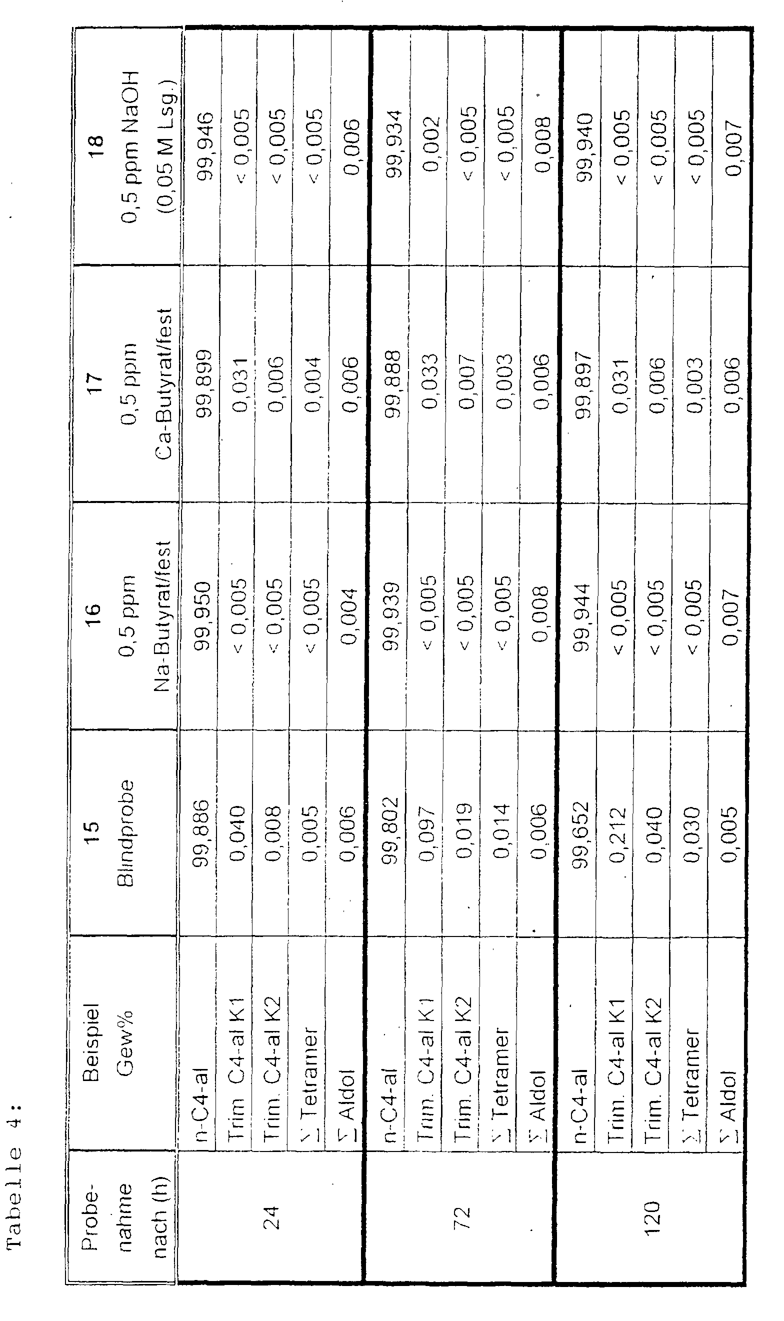

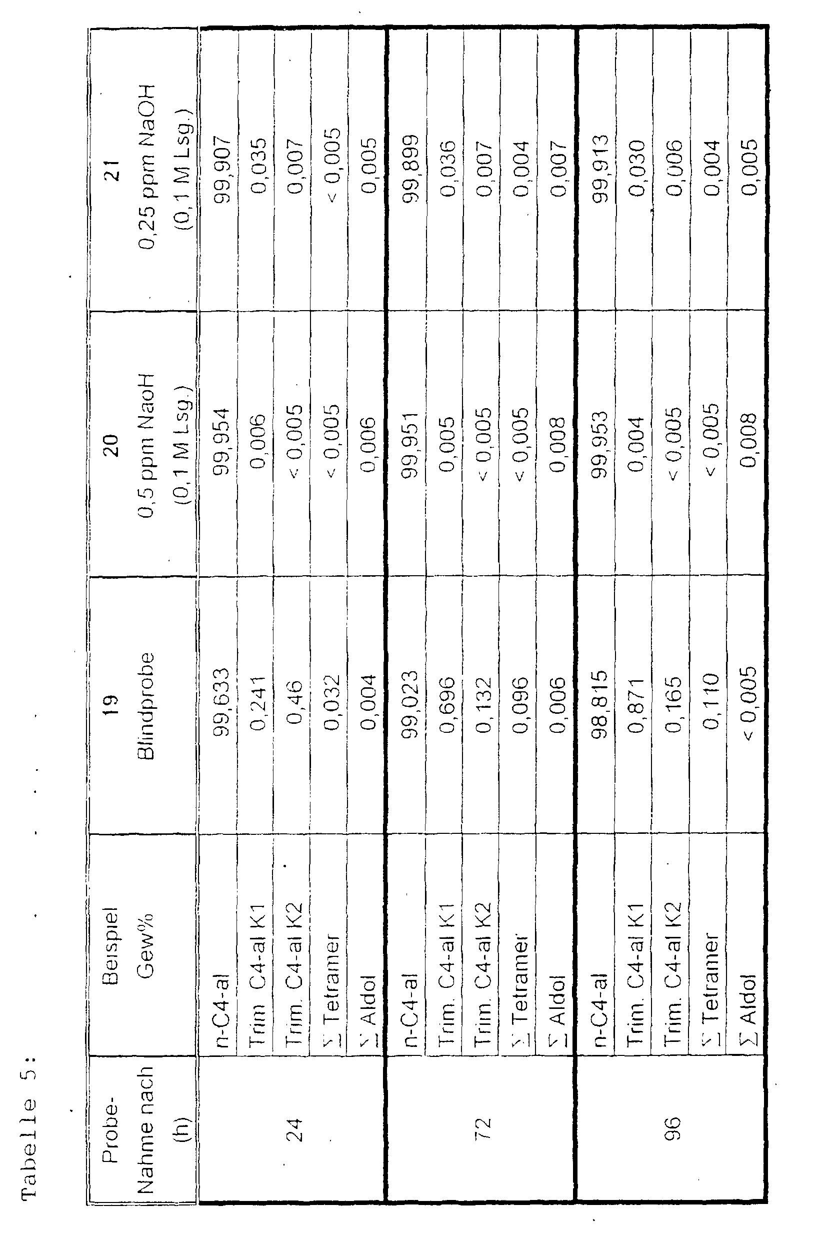

## Patentansprüche

1. Verfahren zur Stabilisierung von aliphatischen C₃-C₁₄-Aldehyden durch den Zusatz von alkalisch reagierenden Substanzen, **dadurch gekennzeichnet, daß** als alkalisch reagierende Substanz Alkalimetallhydroxide, Erdalkalimetallhydroxide, Alkalimetallcarbonate, Erdalkalimetallcarbonate, Alkalimetallcarboxylate oder Erdalkalimetallcarboxylate dem zu stabilisierenden Aldehyd in Mengen von 0.05-20 ppm, bevorzugt 0.05-5 ppm, besonders bevorzugt 0.05-2,5 ppm, bezogen auf den Aldehyd, zugesetzt werden.

2. Verfahren nach Anspruch 1, **dadurch gekennzeichnet, daß** als Alkalimetallhydroxide Natrium- oder Kaliumhydroxid, als Erdalkalimetallhydroxide Calciumhydroxid, als Alkalimetallcarbonate Natrium- oder Kaliumcarbonat, als Erdalkalimetallcarbonate Magnesium- oder Calciumcarbonat oder als Alkalimetallcarboxylat Natriumbutyrat verwendet wird.

3. Verfahren nach Anspruch 1 oder 2, **dadurch gekennzeichnet, daß** die alkalisch reagierende Substanz als 0.01 bis 1 M, bevorzugt 0.05 bis 0.5 M und insbesondere 0.1 bis 0.25 M wäßrige Lösung eingesetzt wird.

4. Verfahren nach einem oder mehreren der Ansprüche 1 bis 3, **dadurch gekennzeichnet, daß** als aliphatische C₃ - C₁₄-Aldehyde Propanal, n- und i-Butanal, n- und i-Pentanal, n- und i-Hexanal, n- und i-Heptanal, n- und i-Octanal, n- und i-Nonanal, n- und i-Decanal, Undecanal, Dodecanal, Laurinaldehyd, Methylnonylaldehyd (MNA), Tridecylaldehyd oder Myristylaldehyd eingesetzt werden.

5. Verfahren nach Anspruch 4, **dadurch gekennzeichnet, daß** die Aldehyde bis zu 3 Gew.%, bevorzugt 0.5 bis 2 Gew.% und insbesondere 0.75 bis 1.25 Gew.% Wasser enthalten.

6. Verfahren nach einem oder mehreren der Ansprüche 1 bis 5, **dadurch gekennzeichnet, daß** die alkalisch reagierende Substanz in Form der wäßrigen Lösung vorgelegt wird und der gegebenenfalls ebenfalls wasserhaltige Aldehyd hinzugegeben wird.

7. Verfahren nach einem oder mehreren der Ansprüch 1 bis 6, **dadurch gekennzeichnet, daß** die wässrige Lösung der alkalisch reagierenden Substanz zu dem gegebenenfalls wasserhaltigen Aldehyd gegeben wird.

## Claims

1. A method of stabilizing aliphatic C₃-C₁₄-aldehydes by addition of alkaline substances, which comprises adding alkali metal hydroxides, alkaline earth metal hydroxides, alkali metal carbonates, alkaline earth metal carbonates, alkaline metal carboxylate or alkaline earth metal carboxylates as alkaline substances to the aldehyde to be stabilized in amounts of 0.05-20 ppm, preferably 0.05-5 ppm, particularly preferably 0.05-2.5 ppm, based on the aldehyde.

2. The method as claimed in claim 1, wherein alkali metal hydroxides used are sodium or potassium hydroxide, the alkaline earth metal hydroxide used is calcium hydroxide, alkali metal carbonates used are sodium or potassium carbonate, alkaline earth metal carbonates used are magnesium or calcium carbonate or the alkali metal carboxylate used is sodium butyrate.

3. The method as claimed in claim 1 or 2, wherein the alkaline substance is used as a 0.01-1M, preferably 0.05-0.5M and in particular 0.1-0.25M, aqueous solution.

4. The method as claimed in one or more of claims 1 to 3, wherein aliphatic C₃-C₁₄-aldehydes used are propanal, n- and i-butanal, n- and i-pentanal, n- and i-hexanal, n- and i-heptanal, n- and i-octanal, n- and i-nonanal, n- and i-decanal, undecanal, dodecanal, lauric aldehyde, methylnonyl aldehyde (MNA), tridecyl aldehyde or myristyl aldehyde.

5. The method as claimed in claim 4, wherein the aldehydes contain up to 3% by weight, preferably from 0.5 to 2% by weight and in particular from 0.75 to 1.25% by weight, of water.

6. The method as claimed in one or more of claims 1 to 5, wherein the alkaline substance is initially charged in the form of the aqueous solution and the aldehyde, which may likewise contain water, is added thereto.

7. The method as claimed in one or more of claims 1 to 6, wherein the aqueous solution of the alkaline substance is added to the anhydrous or water-containing aldehyde.

## Revendications

1. Procédé pour la stabilisation d'aldéhydes aliphatiques en C₃-C₁₄ par addition de substances réagissant de manière alcaline, **caractérisé en ce que** l'on ajoute à l'aldéhyde à stabiliser comme substance réagissant de manière alcaline des hydroxydes de métaux alcalins, des hydroxydes de métaux alcalino-terreux, des carbonates de métaux alcalins, des carbonates de métaux alcalino-terreux, des carboxylates de métaux alcalins ou des carboxylates de métaux alcalino-terreux dans des quantités de 0,05-20 ppm, de préférence de 0,05-5 ppm, encore mieux de 0,05-2,5 ppm, rapportées à l'aldéhyde.

2. Procédé selon la revendication 1, **caractérisé en ce que** l'on utilise comme hydroxydes de métaux alcalins l'hydroxyde de sodium ou de potassium, comme hydroxydes de métaux alcalino-terreux l'hydroxyde de calcium, comme carbonates de métaux alcalins le carbonate de sodium ou de potassium, comme carbonates de métaux alcalino-terreux le carbonate de magnésium ou de calcium ou comme carboxylates de métal alcalin le butyrate de sodium.

3. Procédé selon la revendication 1 ou 2, **caractérisé en ce que** la substance réagissant de manière alcaline est utilisée comme solution aqueuse à de 0,01 à 1 M, de préférence de 0,05 à 0,5 M et en particulier de 0,1 à 0,25 M.

4. Procédé selon l'une ou plusieurs quelconques des revendications 1 à 3, **caractérisé en ce que** l'on utilise comme aldéhydes aliphatiques en C₃-C₁₄ le propanal, le n- et le i-butanal, le n- et le i-pentanal, le n- et le i-hexanal, le n- et le i-heptanal, le n- et le i-octanal, le n- et le i-nonanal, le n- et le undécanal, l'undécanal, le dodécanal, le laurinaldéhyde, le méthylnonylaldéhyde (MNA), le tridécylaldéhyde ou le myristylaldéhyde.

5. Procédé selon la revendication 4, **caractérisé en ce que** les aldéhydes contiennent jusqu'à 3 % en poids, de préférence de 0,5 à 2 % en poids et en particulier de 0,75 à 1,25 % en poids d'eau.

6. Procédé selon l'une ou plusieurs quelconques des revendications 1 à 5, **caractérisé en ce que** la substance réagissant de manière alcaline est présente dans la forme de la solution aqueuse et est ajoutée à l'aldéhyde contenant éventuellement également de l'eau.

7. Procédé selon l'une ou plusieurs quelconques des revendications 1 à 6, **caractérisé en ce que** la solution aqueuse de la substance réagissant de manière alcaline est ajoutée à l'aldéhyde contenant éventuellement de l'eau.
